# FASCICULE DE BREVET EUROPEEN

(11) **EP 0 612 517 B1**
(45) Date de publication et mention de la délivrance du brevet: **07.05.1997**
(21) Numéro de dépôt: 94400353.2
(22) Date de dépôt: 18.02.1994
(51) Int. Cl.: A61K 7/48, A61K 7/00

(54) **Emulsion eau-dans-huile à usage cosmétique ou pharmaceutique**
Wasser in Öl Emulsion zur kosmetischen oder pharmazeutischen Verwendung
Water in oil emulsion for cosmetic or pharmaceutic use

(30) Priorité: 23.02.1993 FR 9302048
(43) Date de publication de la demande: 31.08.1994
(73) Titulaire: L'OREAL, 75008 Paris (FR)
(72) Inventeur: Pastour, Valérie, F-92150 Suresnes (FR); Pouget, Françoise, F-94120 Fontenay Sous Bois (FR); Fodor, Pierre, F-92380 Garches (FR)
(74) Mandataire: Stalla-Bourdillon, Bernard

(56) Documents cités:
- EP-A- 0 374 332
- FR-A- 2 686 510

## Description

La présente invention a pour objet des émulsions eau-dans-huile (E/H) à usage cosmétique ou pharmaceutique ayant une forte teneur en silicone.

De telles émulsions E/H sont utiles en cosmétique notamment pour leur aptitude à former des films à la surface de la peau prévenant efficacement la perte d'eau trans-épidermique et donnant une bonne résistance aux contaminations par les micro-organismes.

Il est connu que plus la teneur en huile de silicone augmente, plus l'obtention d'une émulsion E/H stable est difficile non seulement dans le temps mais également lorsqu'elle est soumise à d'importantes variations de température.

Ce problème de stabilité a pu être au moins partiellement résolu, selon le brevet US 4.698.178, par l'emploi d'une nouvelle classe de tensioactifs siliconés associés à des polyols pour les températures basses et à des électrolytes ou à des savons métalliques pour les températures élevées.

La stabilisation des émulsions E/H a également été réalisée, selon la demande EP 331.833, par l'emploi de tensioactifs siliconés associés à des argiles minérales gonflables à l'eau ou, selon la demande EP 374.332, par l'emploi de cires.

Il a maintenant été découvert de façon inattendue et surprenante que par l'emploi d'une classe de gélifiants particuliers de la phase grasse, il était possible d'obtenir des émulsions E/H ayant non seulement une bonne stabilité dans le temps mais encore vis-à-vis des variations de température.

La présente invention a donc pour objet une émulsion eau-dans-huile stable, à usage cosmétique ou pharmaceutique, constituée d'une phase grasse contenant une silicone, d'une phase aqueuse non gélifiée, d'un agent émulsionnant choisi parmi un alkyl ou alcoxy-diméthicone copolyol ou un diméthicone copolyol, ou leurs mélanges, caractérisée par le fait que la phase grasse représente de 15 à 40 % en poids du poids total de l'émulsion, ladite phase grasse étant constituée d'au moins 10 à 90 % en poids de silicone et de 0,1 à 5 % d'un agent gélifiant constitué d'un mélange d'esters d'acides gras de glycérol et de glycol en un rapport compris entre 75/25 et 95/5 % en poids, lesdits acides gras étant en C₁₆-C₃₆, 50 % au moins desdits acides gras étant en C₁₈-C₂₂.

L'émulsion E/H selon l'invention répond parfaitement aux normes de stabilité soit :
- résistance à l'épreuve de centrifugation à 4000 tr/mn pendant 1 heure,
- résistance au vieillissement à température ambiante pendant 3 mois ainsi qu'à 45°C et à +4°C, et
- résistance à 8 cycles successifs de 8 heures chacun dont les températures s'échelonnent de -20°C à +20°C.

L'émulsion selon l'invention répond aux critères suivants :
- elle a et conserve au cours de ces tests un aspect macroscopique et microscopique homogène et stable (globules finement dispersés, absence de relargage) et
- sa viscosité est constante au cours du temps.

L'émulsion selon l'invention possède par ailleurs de bonnes qualités sensorielles notamment une grande facilité d'application, du confort, de la douceur, une bonne matité, de l'uniformité et de la tenue. Sa préparation est facilitée par l'utilisation de l'agent gélifiant.

Parmi les agents gélifiants qui peuvent être utilisés selon l'invention et qui correspondent à la définition donnée ci-dessus, on peut citer notamment le produit vendu sous la dénomination de "Unitwix" par la Société United Guardian.

Ce produit est essentiellement constitué d'environ 85 % en poids d'esters de glycérol et 15 % en poids d'esters de glycol. Il présente un point de fusion d'environ 68-70°C et la teneur en acides gras en C₁₈-C₂₂ est comprise entre environ 60 et 65 %.

L'agent émulsionnant selon l'invention est un alkyl- ou alcoxy-diméthicone copolyol ou un diméthicone copolyol de formule générale : 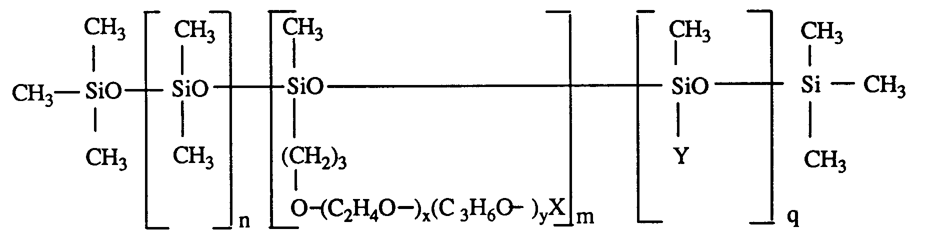 dans laquelle :
X est un atome d'hydrogène, un alkyle, un alcoxy ou un acyle, en C₁-C₁₆,
Y est un radical alkyle ou alcoxy en C₈ à C₂₂,
n = 0 à 200,
m = 1 à 40,
q = 0 à 100,
le poids moléculaire du reste (C₂H₄O-)ₓ(C₃H₆O-)_{y}-X étant de 250 à 2000, x et y étant choisis de telle sorte que le rapport en poids des groupes oxyéthylène/oxypropylène soit compris entre 100:0 et 20:80.

L'agent tensio-actif ou émulsionnant tel que défini ci-dessus est utilisé selon l'invention en une proportion comprise entre 0,5 et 10 % de préférence entre 2 et 6 % en poids par rapport au poids total de l'émulsion et s'est avéré moins irritant que certains autres tensioactifs.

Parmi les produits du commerce pouvant contenir tout ou partie des alkyldiméthicones copolyols utilisables selon l'invention comme émulsionnant on peut citer notamment ceux vendus sous les dénominations de "Abil WE09" ou "Abil WS08" ou "Abil EM90" par la Société Goldschmidt, de "Q2 5200" par la Société Dow Corning et de "218-1138" par la Société General Electric.

Parmi les produits du commerce pouvant contenir tout ou partie des diméthicones copolyols utilisables selon l'invention, on peut citer notamment celui vendu sous la dénomination de "SF1228" par la Société General Electric.

La silicone utilisable selon l'invention peut être un polydiorganosiloxane linéaire, éventuellement fonctionnalisé, ou cyclique ou un organopolysiloxane éventuellement réticulé ou un mélange de ceux-ci.

Les polydiorganosiloxanes linéaires éventuellement fonctionnalisés utilisables selon l'invention répondent à la formule générale suivante : 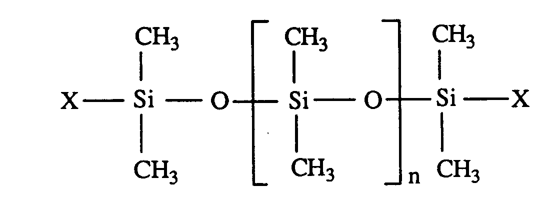 dans laquelle :
X est -CH₃ ou OH, et
n est 0 à 5000.

Parmi ceux-ci, on citera notamment les produits vendus sous la dénomination de "AK" par la Société WACKER, "SF" par la Société GENERAL ELECTRIC et "ABIL" par la Société GOLDSCHMIDT, tel que le produit "Abil 10" ou bien les produits vendus sous la dénomination de "Q2 1401" et "Q2 1403" par la Société DOW CORNING.

Comme polydiorganosiloxanes cycliques selon l'invention, on peut utiliser, seuls ou en mélange, des cyclométhicones de formule : 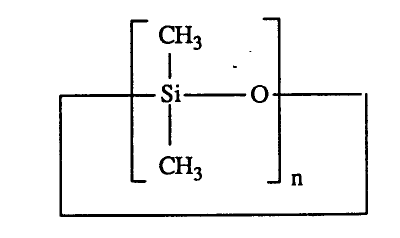 dans laquelle :
n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohexadiméthylsiloxane (n = 6).

On peut notamment utiliser les produits vendus sous les dénominations de "DC Fluid 244", "DC Fluid 245", "DC FLuid 344 et "DC Fluid 345" par la Société Dow Corning.

D'autres cyclométhicones utilisables selon l'invention sont celles vendues sous les dénominations de "Abil K4" par la Société GOLDSCHMIDT ; sous les dénominations de "Silbione 70045 V2" et de "Silbione Huile 70045 V5" par la Société RHONE POULENC; ainsi que sous les dénominations de "Volatil Silicone 7158" et de "Volatil Silicone 7207" par la Société UNION CARBIDE.

Les organopolysiloxanes selon l'invention peuvent être des alkyl, alcoxy ou phényl-diméthicones tels que, par exemple:
(a) une alcoxy diméthicone ayant l'une des formules suivantes : 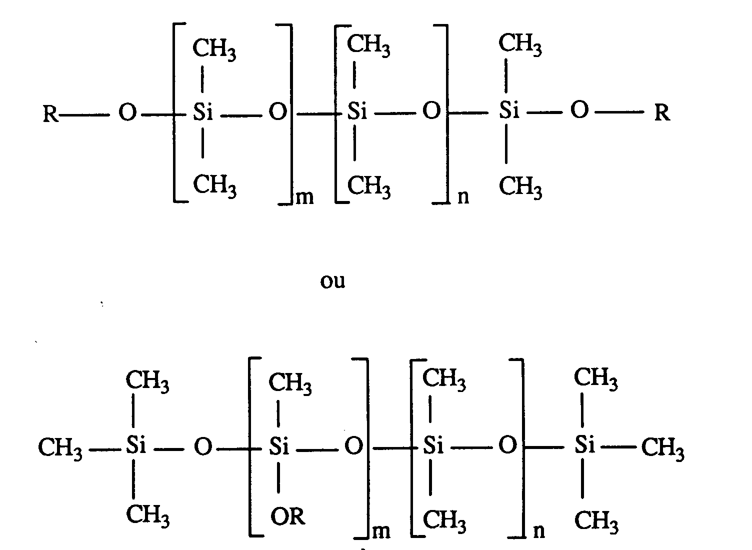 dans lesquelles :
   R est un radical alkyle de C₆ à C₃₀
   m est 1 à 100, et
   n est 0 à 100.

   On peut notamment citer le produit vendu sous la dénomination de "Abil wax 2440" par la Société GOLDSCHMIDT.
(b) une alkyldiméthicone ayant l'une des formules suivantes : 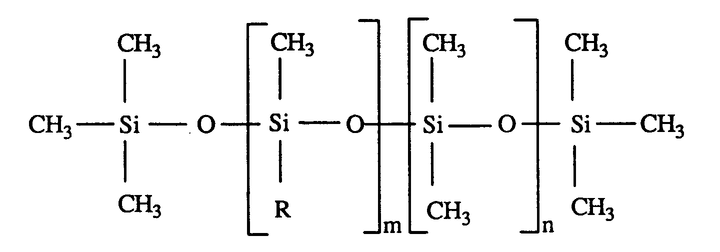 dans laquelle :
   R est un radical alkyle de C₆ à C₃₀,
   m est 1 à 100 et
   n est 0 à 100, ou 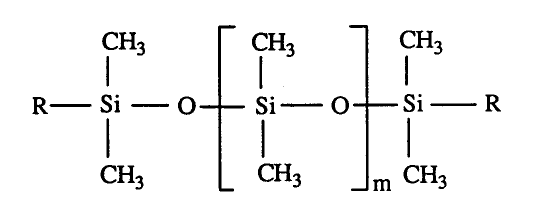
   dans laquelle :
   R est un radical alkyle de C₆ à C₃₀
   m est 1 à 100.
(c) une phényl diméthicone ayant la formule suivante : 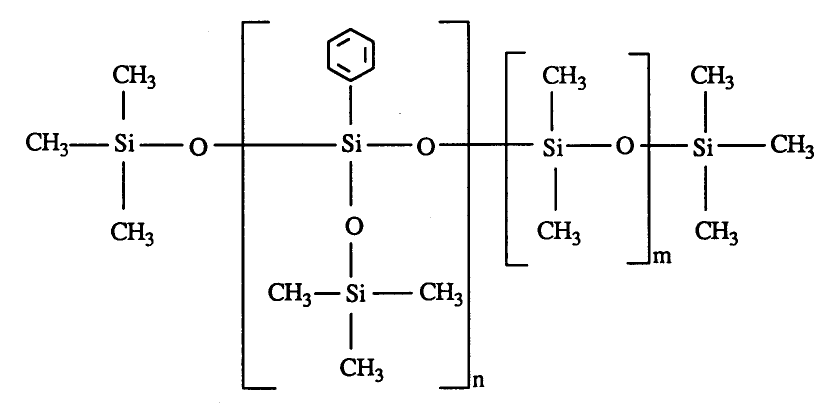 dans laquelle :
   m est 0 à 100 et
   n est 1 à 400
   ou la formule suivante : 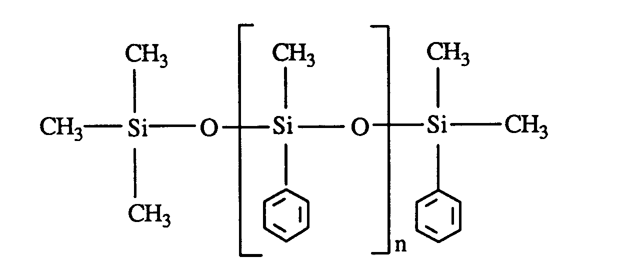 dans laquelle :
   n est 0 à 400.

Les silicones utilisables selon l'invention peuvent être également des résines de silicone comprenant une combinaison des unités :
R₃SiO_{1/2} , R₂SiO_{2/2} , RSiO_{3/2} et SiO_{4/2}

R étant un radical alkyle inférieur en C₁ à C₆ ou phényle.

Tel qu'indiqué ci-dessus, la silicone utilisée selon l'invention est présente en une proportion d'au moins 10 % et de préférence comprise entre 20 et 90 % en poids par rapport au poids de la phase grasse.

La phase grasse de l'émulsion E/H selon l'invention peut comprendre une ou des huile(s) hydrocarbonée(s) dans une proportion comprise entre 0,1 et 52 % en poids par rapport au poids total de la phase grasse de l'émulsion.

Comme huile hydrocarbonée, on citera : toute huile (ou mélange d'huiles) fluide stable à la température d'utilisation habituelle des produits cosmétiques et pharmaceutiquement ou cosmétiquement acceptable telles que les huiles végétales ou animales, les huiles minérales ou synthétiques, les huiles fluorées et les triglycérides d'acides gras en C₁₂-C₁₈.

Parmi les huiles végétales ou animales, modifiées ou non, on peut citer par exemple l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau, l'huile de calophyllum.

Parmi les huiles minérales, on peut citer par exemple l'huile de vaseline.

Parmi les huiles synthétiques, on peut citer notamment les isoparaffines et les poly-isobutènes.

Selon un mode de réalisation particulier, l'émulsion selon l'invention peut également comprendre un tensioactif additionnel tel qu'un ester ou un éther de glycérol et/ou une dispersion de polydiméthylsiloxane oxyéthyléné dans un cyclodiméthylsiloxane ("Q3225C" de la Société Dow Corning) ayant une HLB comprise entre 2 et 7 et présent en une proportion comprise entre 0,01 et 5 % en poids par rapport au poids total de l'émulsion.

Parmi les tensioactifs additionnels de ce type, on peut citer les esters ou éthers de glycérol notamment l'ester d'acide isostéarique et/ou d'acide succinique et l'éther d'alcool décyltétradécylique. On peut citer par exemple le produit vendu par la Société Hüls sous la dénomination de "Imwitor 780K" qui est un isostéaryl diglycéryl succinate.

Il est par ailleurs disponible dans le commerce, certains produits constitués d'un mélange d'alkyldiméthicone copolyol de la formule donnée ci-dessus et d'un tensioactif additionnel du type mentionné ci-dessus.

On citera à cet égard le produit vendu par la Société Goldschmidt sous la dénomination "Abil WE09" qui contient un alkyldiméthicone copolyol comportant un rapport en poids des groupes oxyéthylène/oxypropylène compris entre 100:0 et 20:80 associé à un isostéarate de glycérol et du laurate d'hexyle.

Selon l'invention, la phase grasse peut également renfermer des pigments éventuellement enrobés par des substances hydrophiles ou hydrophobes tels que :
- le polyéthylène,
- la lécithine,
- un sel d'un acide aminé tel que l'acylglutamate d'aluminium,
- le polyméthylméthacrylate,
- le triisostéaroyltitanate, et
- le collagène.

Parmi les pigments enrobés, on peut citer notamment les pigments vendus sous la dénomination de "Covasil" par la Société WACKER (pigments au triisostéaroyl-titanate).

Les pigments ainsi enrobés peuvent être incorporés dans l'émulsion selon l'invention en une proportion comprise entre 0,1 et 15 % en poids par rapport au poids total de l'émulsion.

Parmi les autres adjuvants liposolubles que l'on peut incorporer à la phase grasse, on peut citer les filtres U.V. lipophiles, les vitamines lipophiles, les antioxydants et les parfums, les céramides.

La phase aqueuse peut également renfermer des adjuvants communément utilisés dans les émulsions E/H cosmétiques. On citera par exemple les lubrifiants, les agents hydratants, tels que le glycérol et le propylène glycol, les oligo-éléments, les filtres UV hydrophiles, les polysaccharides ainsi que des électrolytes tels que NaCl ou MgSO₄. Elle peut également comprendre des principes actifs tels que des extraits végétaux, des extraits bactériens, des protéines ou leurs hydrolysats et notamment des hydrolysats de collagène ou d'élastine.

Ces principes actifs sont alors présents en une proportion comprise entre 1 et 15 %.

L'émulsion selon l'invention peut également incorporer des charges d'origine végétale, minérale ou synthétique, en particulier la poudre d'amidon, de la silice colloïdale, de la poudre de nylon (Orgasol), et du talc.

Les émulsions selon l'invention peuvent être sous forme de crème blanche ou teintée, sous forme de lait, sous forme de fond de teint, de mascara, de blush ou d'un produit de maquillage pour les lèvres.

Le procédé de préparation des émulsions selon l'invention consiste : (a) en un premier temps, à chauffer la phase grasse contenant l'émulsionnant et l'agent gélifiant jusqu'à une température suffisante pour fondre tous les constituants, de préférence entre 60 et 85°C puis à y incorporer les adjuvants liposolubles additionnels éventuels, et (b) en un deuxième temps, après refroidissement de la phase grasse entre 40 et 60°C, à ajouter la phase aqueuse, portée à la même température, à la phase grasse sous faible agitation et de manière lente, puis lorsque la température est revenue à environ 25°C, à soumettre alors la préparation à une forte agitation.

Cette deuxième étape peut être également réalisée par addition sous vive agitation de la phase aqueuse à la phase grasse, la phase aqueuse étant portée à la même température que la phase grasse.

On va maintenant donner à titre d'illustration, plusieurs exemples de compositions cosmétiques sous forme d'émulsions E/H.

### EXEMPLE 1 : Fond de teint

| *Phase grasse A :* | |
|---|---|
| - Agent gélifiant "Unitwix" de la Société United Guardian | 1,5 % |
| - Alkyl diméthicone copolyol "Abil EM90" de la Société Goldschmidt | 2,5 % |
| - Isostéaryl diglycéryl succinate "Imwitor 780K" de la Société Hüls | 3 % |
| - Polyisobutène hydrogéné | 2,5 % |
| - Huile de jojoba | 2 % |
| - Diméthicone "Abil 10" de la Société Goldschmidt | 5 % |
| - Cyclométhicone "7158" de la Société Union Carbide | 8 % |
| - Pigments | 6% |
| - Vitamine E | 0,5 % |

| *Phase aqueuse B :* | |
|---|---|
| - Conservateurs | 0,5 % |
| - NaCl | 1,1 % |
| - Glycérine | 2 % |
| - Silice sphérique | 3,5 % |
| - Eau | qs 100% |

On obtient ce fond de teint en chauffant séparément les phases A et B à 80°C, puis on verse doucement la phase B, dans la phase A sous vive agitation.

Le fond de teint obtenu est d'un bel aspect et présente de bonnes propriétés cosmétiques.

### EXEMPLE 2 : Fond de teint

| *Phase grasse A :* | |
|---|---|
| - Agent gélifiant "Unitwix" de la Société United Guardian | 1,5 % |
| - Alkyldiméthicone copolyol "Q25200" de la Société Dow Corning | 1 % |
| - Alkyldiméthicone "Abil EM90" de la Société Goldschmidt | 2 % |
| - Huile minérale | 2,5 % |
| - Triglycérides d'acides gras en C₁₀-C₁₈ | 7,5 % |
| - Tocophérol | 0,1 % |
| - Cyclométhicone "7158" de la Société Union Carbide | 6 % |
| - Mélange de diméthicone et diméthiconol "Q2 1403" de la Société Dow Corning | 2,5 % |
| - Pigments | 6 % |

| *Phase aqueuse B :* | |
|---|---|
| - Conservateurs | 0,5 % |
| - Glycérine | 4 % |
| - MgSO₄ | 0,8 % |
| - Poudre d'amidon | 2,5 % |
| - Eau | qs 100 % |

On obtient ce fond de teint de la même manière qu'à l'exemple 1.

### EXEMPLE 3 : Crème

| *Phase A :* | |
|---|---|
| - Agent gélifiant "Unitwix" de la Société United Guardian | 2 % |
| - Silicone "218-1138" de la Société General Electric | 4 % |
| - Cyclométhicone "7158" de la Société Union Carbide | 10 % |
| - Diméthicone "Abil 10" de la Société Goldschmidt | 10 % |

| *Phase B :* | |
|---|---|
| - Conservateurs | 0,5 % |
| - Glycérine | 2 % |
| - MgSO₄ | 0,8 % |
| - Eau | qs 100 % |

On obtient cette crème de la même manière que décrite à l'exemple 1.

La crème obtenue est d'un aspect brillant, légèrement translucide, de toucher doux, non collant et frais.

### EXEMPLE 4 : Crème

| *Phase A :* | |
|---|---|
| - Agent gélifiant "Unitwix" de la Société United Guardian | 2 % |
| - Diméthicone copolyol "SF 1228" de la Société General Electric | 1,1 % |
| - Isostéaryl diglycéryl succinate "Imwitor 780K" de la Société Hüls | 3 % |
| - Diméthicone "Abil 10" de la Société Goldschmidt | 7,5 % |
| - Cyclométhicone "7158" de la Société Union Carbide | 7,5 % |
| - Mélange de diméthicone et diméthiconol "Q2 1403" de la Société Dow Corning | 3,5 % |

| *Phase B :* | |
|---|---|
| - Conservateurs | 0,5 % |
| - Glycérine | 4 % |
| - MgSO₄ | 0,7 % |
| - Extrait bactérien "Vitacell" de la Société LSN | 5 % |
| - Eau | qs 100 % |

On obtient cette crème selon le même mode opératoire que décrit à l'exemple 1.

### EXEMPLE 5 : Crème

| *Phase A :* | |
|---|---|
| - Agent gélifiant "Unitwix" de la Société United Guardian | 2 % |
| - Alkyl diméthicone copolyol "Abil EM90" de la Société Goldschmidt | 2,5 % |
| - Perfluoropolyéther "Fomblin HC25" de la Société Montefluos | 1,5 % |
| - Isostéaryl diglycéryl succinate "Imwitor 780K" de la Société Hüls | 3 % |
| - Triméthylsiloxysilicate | 1,5 % |
| - Octyl cocoate | 4 % |
| - Diméthicone "Abil 10" de la Société Goldschmidt | 10 % |

| *Phase B :* | |
|---|---|
| - Extrait bactérien "Oxylastil" de la Société Sederma | 11 % |
| - Conservateurs | 0,5 % |
| - Propylène glycol | 4 % |
| - NaCl | 1 % |
| - Eau | qs 100 % |

On obtient l'émulsion sous forme de crème par chauffage de la phase A à 80°C, puis on ajoute lentement la phase B non chauffée sous faible agitation. Le prémélange étant effectué, on ramène la température à 25°C puis on soumet alors le mélange à une vive agitation pendant 10 minutes.

## Revendications

1. Emulsion eau-dans-huile stable, à usage cosmétique ou pharmaceutique, constituée d'une phase grasse contenant une silicone, d'une phase aqueuse non gélifiée et d'un agent émulsionnant choisi parmi un alkyl ou alcoxydiméthicone copolyol ou un diméthicone copolyol ou leur mélange, caractérisée par le fait que la phase grasse représente de 15 à 40 % en poids du poids total de l'émulsion, ladite phase grasse étant constituée d'au moins 10 à 90% en poids de silicone et de 0,1 à 5 % d'un agent gélifiant constitué d'un mélange d'esters d'acides gras de glycérol et de glycol en un rapport compris entre 75/25 et 95/5 % en poids, lesdits acides gras étant en C₁₆-C₃₆, 50 % au moins desdits acides gras étant en C₁₈-C₂₂.

2. Emulsion selon la revendication 1, caractérisée par le fait que lesdits alkyl ou alcoxy diméthicone copolyols ou diméthicone copolyols répondent à la formule : 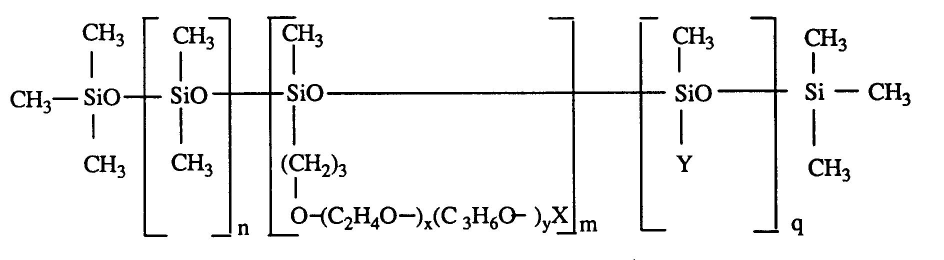 dans laquelle :
X est un atome d'hydrogène, un alkyle, un alcoxy ou un acyle, en C₁-C₁₆,
Y est un radical alkyle ou alcoxy en C₈ à C₂₂,
n = 0 à 200,
m = 1 à 40,
q = 0 à 100,
le poids moléculaire du reste (C₂H₄O-)ₓ(C₃H₆O-)_{y}-X étant de 250 à 2000, x et y étant choisis de telle sorte que le rapport en poids des groupes oxyéthylène/oxypropylène soit compris entre 100:0 et 20:80.

3. Emulsion selon l'une quelconque des revendications précédentes, caractérisée par le fait que l'alkyl- ou alcoxydiméthicone copolyol ou le diméthicone copolyol est présent en une proportion comprise entre 0,5 et 10 % et de préférence entre 2 et 6 % en poids par rapport au poids total de l'émulsion.

4. Emulsion selon la revendication 1, caractérisée par le fait que la silicone est un polydiorganosiloxane linéaire, éventuellement fonctionnalisé, ou cyclique, ou un organopolysiloxane éventuellement réticulé ou un mélange de ceux-ci.

5. Emulsion selon la revendication 4, caractérisée par le fait que le polydiorganosiloxane linéaire répond à la formule : 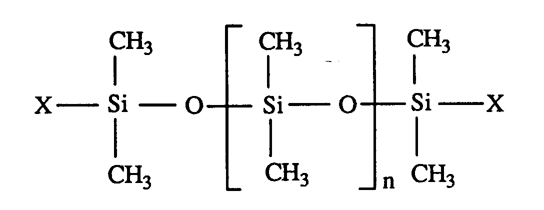 dans laquelle :
X est -CH₃ ou OH et n = 0 à 5000
et que le polydiorganosiloxane cyclique répond à la formule : 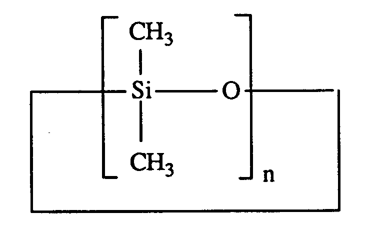 dans laquelle :
n est un nombre entier de 3 à 8.

6. Emulsion selon la revendication 4, caractérisée par le fait que l'organopolysiloxane est choisi dans le groupe constitué par les alkyl, alcoxy et phényl-diméthicones.

7. Emulsion selon l'une quelconque des revendications précédentes, caractérisée par le fait que la phase grasse est en outre constituée d'une huile hydrocarbonée en une proportion comprise entre 0,1 et 52 % en poids par rapport au poids total de la phase grasse.

8. Emulsion selon l'une quelconque des revendications précédentes, caractérisée par le fait qu'elle comprend comme tensioactif supplémentaire un ester ou éther de glycérol et/ou une dispersion de polydiméthylsiloxane oxyéthyléné dans un cyclodiméthylsiloxane ayant une HLB comprise entre 2 et 7 présent en une proportion comprise entre 0,01 et 5 % en poids par rapport au poids total de l'émulsion.

9. Emulsion selon l'une quelconque des revendications précédentes, caractérisée par le fait que la phase grasse comprend en outre au moins un adjuvant liposoluble choisi parmi les filtres U.V. lipophiles, les vitamines lipophiles, les antioxydants et les parfums.

10. Emulsion selon l'une quelconque des revendications précédentes, caractérisée par le fait que la phase aqueuse comprend en outre au moins une substance hydrosoluble choisie parmi les hydratants, les lubrifiants, les polysaccharides, les électrolytes, les filtres U.V. hydrophiles, les oligo-éléments et les principes actifs.

11. Emulsion selon l'une quelconque des revendications précédentes, caractérisée par le fait qu'elle renferme des pigments éventuellement enrobés par des substances hydrophiles ou hydrophobes.

## Claims

1. Stable water-in-oil emulsion, for cosmetic or pharmaceutical use, comprising a fatty phase containing a silicone, a non-gelled aqueous phase and an emulsifying agent chosen from an alkyl- or alkoxydimethicone copolyol or a dimethicone copolyol or their mixture, characterized in that the fatty phase represents from 15 to 40% by weight of the total weight of the emulsion, the said fatty phase comprising at least 10 to 90% by weight of silicone and 0.1 to 5% of a gelling agent comprising a mixture of fatty acid esters of glycerol and of glycol in a ratio of between 75/25 and 95/5% by weight, the said fatty acids being C₁₆-C₃₆ and at least 50% of the said fatty acids being C₁₈-C₂₂.

2. Emulsion according to Claim 1, characterized in that the said alkyl- or alkoxydimethicone copolyols or dimethicone copolyols correspond to the formula: 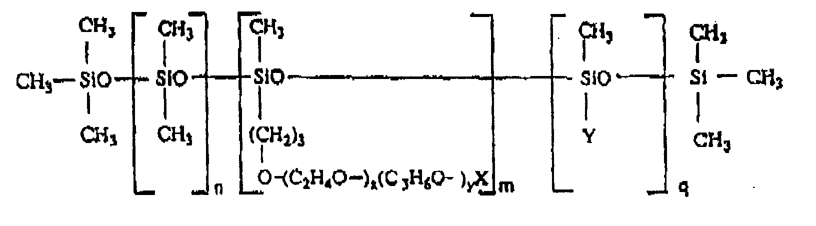 in which:
X is a hydrogen atom or a C₁-C₁₆ acyl, alkoxy or alkyl,
Y is a C₈ to C₂₂ alkoxy or alkyl radical,
n = 0 to 200,
m = 1 to 40,
q = 0 to 100,
the molecular weight of the (C₂H₄O-)ₓ(C₃H₆O-)_{y}-X residue being from 250 to 2000, x and y being chosen so that the ratio by weight of the oxyethylene/oxypropylene groups is between 100:0 and 20:80.

3. Emulsion according to either of the preceding claims, characterized in that the alkyl- or alkoxydimethicone copolyol or dimethicone copolyol is present in a proportion of between 0.5 and 10%, and preferably between 2 and 6%, by weight with respect to the total weight of the emulsion.

4. Emulsion according to Claim 1, characterized in that the silicone is a cyclic or, optionally functionalized, linear polydiorganosiloxane or an optionally crosslinked organopolysiloxane or a mixture of these.

5. Emulsion according to Claim 4, characterized in that the linear polydiorganosiloxane corresponds to the formula: 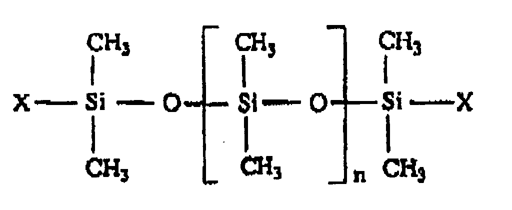 in which:
X is -CH₃ or OH and n = 0 to 5000
and in that the cyclic polydiorganosiloxane corresponds to the formula: 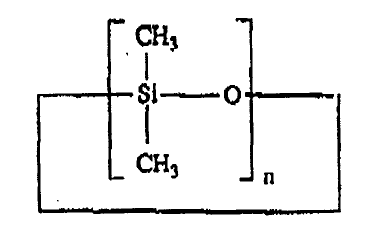 in which:
n is an integer from 3 to 8.

6. Emulsion according to Claim 4, characterized in that the organopolysiloxane is chosen from the group consisting of alkyl-, alkoxy- and phenyldimethicones.

7. Emulsion according to any one of the preceding claims, characterized in that the fatty phase additionally comprises a hydrocarbon oil in a proportion of between 0.1 and 52% by weight with respect to the total weight of the fatty phase.

8. Emulsion according to any one of the preceding claims, characterized in that it comprises, as additional surface-active agent, a glycerol ether or ester and/or an oxyethylenated polydimethylsiloxane dispersion in a cyclodimethylsiloxane, having an HLB of between 2 and 7, present in a proportion of between 0.01 and 5% by weight with respect to the total weight of the emulsion.

9. Emulsion according to any one of the preceding claims, characterized in that the fatty phase additionally comprises at least one liposoluble adjuvant chosen from lipophilic U.V. screening agents, lipophilic vitamins, antioxidizing agents and fragrances.

10. Emulsion according to any one of the preceding claims, characterized in that the aqueous phase additionally comprises at least one water-soluble substance chosen from hydrating agents, lubricating agents, polysaccharides, electrolytes, hydrophilic U.V. screening agents, trace elements and active principles.

11. Emulsion according to any one of the preceding claims, characterized in that it contains pigments optionally coated with hydrophilic or hydrophobic substances.

## Patentansprüche

1. Stabile Wasser-in-Öl-Emulsion für die kosmetische oder pharmazeutische Verwendung, wobei die Emulsion aus einer Fettphase mit Gehalt an einem Silikon, einer nichtgelierten wäßrigen Phase und einem Emulgator besteht, der unter einem Alkyl- oder Alkoxydimethiconcopolyol oder einem Dimethiconcopolyol oder einem Gemisch daraus besteht, dadurch gekennzeichnet, daß die Fettphase 15 bis 40 Gew.-% in Bezug auf das Gesamtgewicht der Emulsion ausmacht, wobei die Fettphase aus mindestens 10 bis 90 Gew.-% Silikon und 0,1 bis 5 Gew.-% Geliermittel besteht, das wiederum aus einem Gemisch aus Fettsäurestern des Glycerins und Glykols in einem Mengenverhältnis besteht, das zwischen 75/25 und 95/5 Gew.-% beträgt und wobei es sich um C₁₆-C₃₆ Fettsäuren handelt, wovon mindestens 50 % C₁₈-C₂₂ Fettsäuren darstellen.

2. Emulsion nach Anspruch 1 dadurch gekennzeichnet, daß die Alkyl- oder Alkoxydimethiconcopolyole oder die Dimethiconcopolyole der folgenden Formel entsprechen: 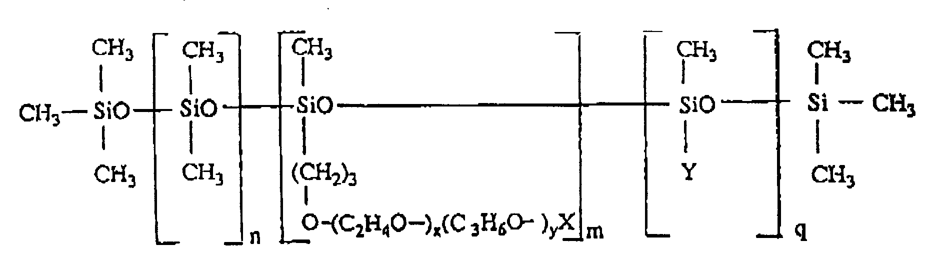 worin
X ein Wasserstoffatom, einen C₁-C₁₆-Alkyl-, -Alkoxy- oder -Acylrest darstellt,
Y einen C₈-C₂₂-Alkyl- oder -Alkoxyrest bedeutet,
n = 0 bis 200,
m = 1 bis 40 und
q = 0 bis 100 bedeuten, wobei
das Molekulargewicht des Restes (C₂H₄O-)ₓ(C₃H₆O-)_{y}-X 250 bis 2000 beträgt und x und y in der Weise ausgewählt sind, daß das Gewichtsverhältnis der Oxyethylen/Oxypropylen-Gruppen zwischen 100 : 0 und 20 : 80 beträgt.

3. Emulsion nach einem der vorstehenden Ansprüche, dadurch gekennzeichnet, daß das Alkyl- oder Alkoxydimethiconcopolyol oder das Dimethiconcopolyol in einem Mengenverhältnis zwischen 0,5 und 10 Gew.-%, vorzugsweise zwischen 2 und 6 Gew.-% in Bezug auf das Gesamtgewicht der Emulsion vorhanden ist.

4. Emulsion nach Anspruch 1, dadurch gekennzeichnet, daß das Silikon ein lineares, gegebenenfalls funktionalisiertes oder cyclisches Polydiorganosiloxan oder ein gegebenenfalls vernetzes Organopolysiloxan bzw. deren Gemisch ist.

5. Emulsion nach Anspruch 4, dadurch gekennzeichnet, daß das lineare Polydiorganosiloxan der folgenden Formel entspricht: 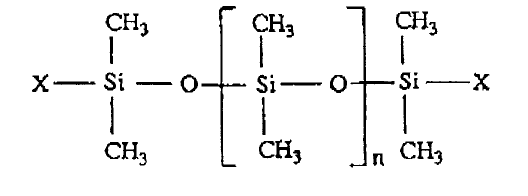 worin:
X -CH₃ oder OH und n = 0 bis 5000 bedeuten und
das cyclische Polydiorganosiloxan der folgenden Formel entspricht: 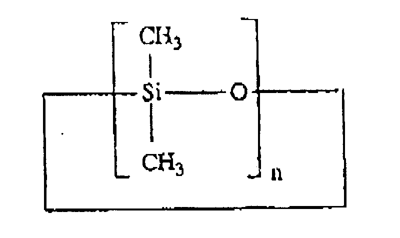 worin:
n eine ganze Zahl von 3 bis 8 bedeutet.

6. Emulsion nach Anspruch 4, dadurch gekennzeichnet, daß das Organopolysiloxan aus der aus Alkyl-, Alkoxy- und Phenyldimethiconen bestehenden Gruppe ausgewählt ist.

7. Emulsion nach einem der vorstehenden Ansprüche, dadurch gekennzeichnet, daß die Fettphase zusätzlich noch aus einem Kohlenwasserstoffol besteht, das in einem Mengenverhältnis zwischen 0,1 und 52 Gew.-% in Bezug auf das Gesamtgewicht der Fettphase vorhanden ist.

8. Emulsion nach einem der vorstehenden Ansprüche, dadurch gekennzeichnet, daß sie als zusätzliches Tensid einen Ester oder Ether des Glycerins und/oder eine Dispersion aus einem oxyethylierten Polydimethylsiloxan in einem Cyclodimethylsiloxan enthält, das einen HLB-Wert aufweist, der zwischen 2 und 7 beträgt,und wobei diese Dispersion in einem Mengenverhältnis zwischen 0,01 und 5 Gew.-% in Bezug auf das Gesamtgewicht der Emulsion vorhanden ist.

9. Emulsion nach einem der vorstehenden Ansprüche, dadurch gekennzeichnet, daß die Fettphase zusätzlich noch einen fettlöslichen Hilfsstoff umfaßt, der aus lipophilen UV-Flitersubstanzen, lipophilen Vitaminen, Antioxidantien und Duftstoffen ausgewählt ist.

10. Emulsion nach einem der vorstehenden Ansprüche, dadurch gekennzeichnet, daß die wäßrige Phase zusätzlich noch mindestens eine wasserlösliche Substanz enthält, die aus Hydratisierungsmitteln, Gleitmitteln, Polysacchariden, Elektrolyten, hydrophilen UV-Filtersubstanzen und Spurenelementen sowie Wirkstoffen ausgewählt ist.

11. Emulsion nach einem der vorstehenden Ansprüche, dadurch gekennzeichnet, daß sie gegebenenfalls mit hydrophilen oder hydrophoben Substanzen überzogene Pigmente umfaßt.
